(19) Europäisches Patentamt
European Patent Office
Office européen des brevets

(11) Publication number: **0 015 776**
**B1**

(12) # EUROPEAN PATENT SPECIFICATION

(45) Date of publication of patent specification: **30.06.82**

(21) Application number: **80300744.2**

(22) Date of filing: **11.03.80**

(51) Int. Cl.³: **C 07 C 91/16,**
C 07 D 295/08,
C 07 C 87/28,
C 07 C 103/18,
C 07 C 101/30,
C 07 C 91/26,
C 07 C 121/66,
A 61 K 31/135,
A 61 K 31/16,
A 61 K 31/195,
A 61 K 31/275 //C07C97/10,
C07C87/29

(54) **1,1'-Biphenyl-2-yl alkylamine derivatives, their production and cardiovascular preparations containing same.**

(30) Priority: **12.03.79 US 19534**

(43) Date of publication of application:
**17.09.80 Bulletin 80/19**

(45) Publication of the grant of the patent:
**30.06.82 Bulletin 82/26**

(84) Designated Contracting States:
**DE GB NL SE**

(56) References cited:
**CH - A - 364 264**
**GB - A - 1 475 314**
**GB - A - 1 476 106**
**GB - A - 1 523 655**
**US - A - 3 433 836**

**J. MED. CHEM. VOL. 10, May 1967, Pages 489—491**
**Washington, D.C., USA**
**G. TSATSAS et al.: "Synthesis of Substituted 2-(2-Biphenylyl) ethylamines as Potential Analgetics"**

(73) Proprietor: **ELI LILLY AND COMPANY**
**307, East McCarty Street**
**Indianapolis Indiana 46285 (US)**

(72) Inventor: **Lacefield, William Bryant**
**636, Boulder Road**
**Indianapolis Indiana (US)**
Inventor: **Simon, Richard Lee**
**371 Redbud Place**
**Greenwood Indiana (US)**

(74) Representative: **McVey, Kenneth William Henry et al,**
**Lilly House 13 Hanover Square**
**London W1R 0PA (GB)**

**EP 0 015 776 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

1,1′-Biphenyl-2-yl alkylamine derivatives, their production and cardiovascular preparations containing same

This invention relates to a novel class of 1,1′-biphenyl-2-yl alkylamine derivatives which are useful in the chemotherapy of cardiovascular disorders.

Cardiovascular disorders are responsible for thousands of deaths each year. Cardiac arrhythmias are one of these disorders contributing to such deaths. Whilst the true causes of arrhythmias are unknown, it is believed that they are caused by some abnormality in the rate, regularity or site of origin of cardiac impulses, or by disturbances which affect the sequence of activation of the atria and ventricles.

Several drugs currently are used in the treatment of arrhythmias, the most notable including quinidine, procainamide, lidocaine and digitalis. A great deal of interest recently has been generated over a new antiarrhythmic agent, aprindine, a 2-aminoindane derivative; see U.S. Patent No. 3,923,813. Several diphenylalkylamines also have recently been reported to have useful antiarrhythmic activity; see U.S. Patent Nos. 4,001,328, 4,034,011 and 3,987,201.

Unfortunately, most if not all of these existing drugs possess undesirable side effects and thus there exists a continuing need for new drugs capable of being used in the treatment of arrythmias and possessing reduced incidence of undesired side effects.

Accordingly, the present invention provides compounds of formula (I):

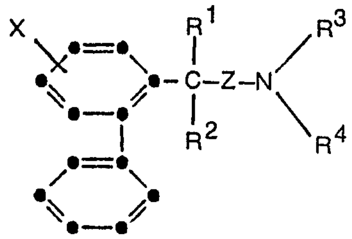

(I)

wherein:

X is hydrogen or a halo substituent at any position in the phenyl ring;

$R^1$ is hydrogen, hydroxy, C≡N, $CONR^5R^6$, where $R^5$ and $R^6$ independently represent hydrogen or $C_{1-6}$ alkyl, or $CO_2R^8$ where $R^8$ is hydrogen or $C_{1-4}$ alkyl;

$R^2$ is hydrogen or $C_{1-3}$ alkyl;

$$Z \text{ is } -(CH_2)_n- \text{ or } -CH_2\overset{OH}{\overset{|}{C}}HCH_2-;$$

where n is an integer of from 2 to 4;

$R^3$ and $R^4$ independently are hydrogen, $C_{1-6}$ alkyl, $CH_2C_{2-5}$ alkenyl, phenyl $C_{1-3}$ alkyl, or taken together with the adjacent nitrogen atom form a heterocyclic group containing from 5 to 7 ring atoms and optionally containing one heteroatom selected from oxygen and nitrogen;

and the pharmaceutically-acceptable salts thereof.

Preferred compounds of formula (I) are those wherein X is hydrogen, or fluoro, chloro or bromo situated at the 5-position of the biphenylyl ring; $R^1$ is hydrogen, hydroxy, C≡N, $CONH_2$ or COOH; $R^2$ is hydrogen or methyl, Z is $-(CH_2)_n-$ where n is 2 or 3 or is $-CH_2CH(OH)CH_2-$ when $R^1$ is hydrogen; and $R^3$ and $R^4$ independently are hydrogen, $C_{1-6}$ alkyl, $CH_2C_{2-5}$ alkenyl, phenyl $C_{1-3}$ alkyl, or taken together with the adjacent nitrogen atom form a heterocyclic group selected from pyrrolidino, piperidino and morpholino; and the pharmaceutically-acceptable acid addition or quaternary ammonium salts thereof.

Other preferred compounds of formula (I) have one or more of the following features:

1. X is hydrogen or fluoro;
2. $R^1$ is hydroxy;
3. $R^1$ is C≡N;
4. $R^1$ is $CONH_2$;
5. $R^2$ is hydrogen;
6. $R^3$ is other than hydrogen;
7. $R^3$ is $C_{1-4}$ alkyl, especially *iso*propyl;
8. $R^4$ is hydrogen or $C_{1-4}$ alkyl;
9. Z is $-CH_2CH_2CH_2-$;
10. $Z \text{ is } -CH_2-\overset{OH}{\overset{|}{C}}HCH_2-;$

11. are pharmaceutically-acceptable acid addition salts; or

12. are pharmaceutically-acceptable quaternary ammonium salts.

This invention also provides pharmaceutical formulations which comprise a compound of formula (I), or a pharmaceutically-acceptable salt thereof, associated with a pharmaceutically-acceptable carrier therefor.

The biphenylyl alkylamines provided by the invention are basic substances which readily react with organic and inorganic acids to form salts. Those acid addition salts which are substantially as nontoxic as the free amine from which they are derived are "pharmaceutically-acceptable acid addition salts" and are provided as a further embodiment of the invention. Such salts are those prepared with common inorganic acids such as hydrochloric acid, hydrobromic acid, sulfuric acid, phosphoric acid or perchloric acid. Pharmaceutically-acceptable acid addition salts can also be prepared by reaction of the amines with organic acids such as formic acid, acetic acid, succinic acid, maleic acid, *para*-toluenesulfonic acid or methanesulfonic acid, citric acid. Pharmaceutically-acceptable salts also include the quaternary ammonium salts of amine bases of the formula (I). Such salts can be prepared by reaction of a biphenylyl alkyl tertiary amine of the invention with an alkylating agent such as a $C_{1-6}$ alkyl halide, $C_{1-6}$ alkyl sulfate, or $C_{1-6}$ alkyl benzene sulfate. Typical alkylating agents commonly used to form acceptable quaternary ammonium salts include methyl bromide, *n*-butyl iodide, *iso*pentyl chloride, dimethylsulfate, ethyl benzenesulfate and diisopropylsulfate.

The compounds of formula (I) may be prepared by:

(a) reacting a ketone of formula (II)

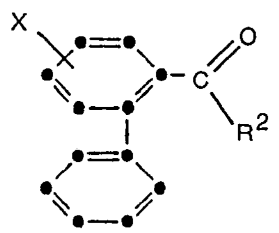

(II)

with a Grignard reagent of formula:

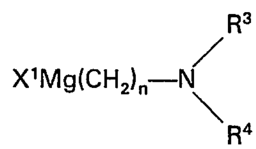

where $X^1$ is chloro, bromo or iodo and n is 3 or 4, to produce a compound of formula (I) in which $R^1$ is hydroxy and Z is $-(CH_2)_n-$ where n is 3 or 4;

(b) reducing a compound of formula (II)

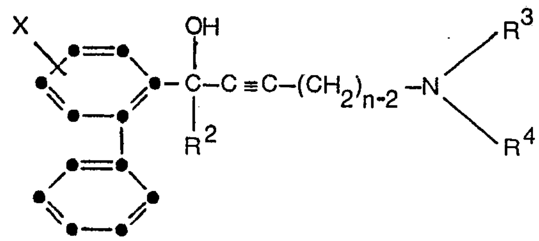

(II)

to form a compound of formula (I) where $R^1$ is hydroxy and Z is $-(CH_2)_n-$ where n is 3 or 4;

(c) reducing a ketone of formula (III)

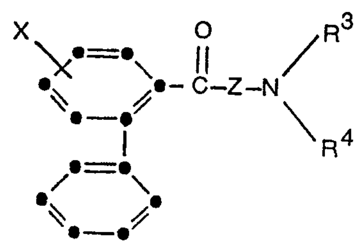

(III)

to form a compound of formula (I) in which $R^1$ is hydroxy and $R^2$ is hydrogen;

(d) alkylating a nitrile of formula (IV);

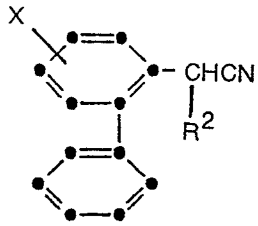

(IV)

with an aminoalkylating agent of formula:

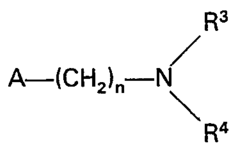

where A is a leaving group; to form a compound of formula (I) where $R^1$ is —C≡N;

(e) hydrolyzing a nitrile of formula (V):

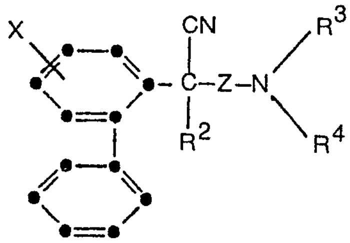

(V)

to form a compound of formula (I) in which $R^1$ is $—CONH_2$ or —COOH;

(f) condensing an epoxide of formula (VI)

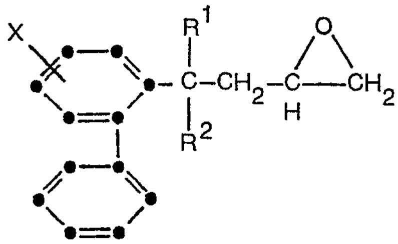

(VI)

with an amine of formula $HNR^3R^4$, where $R^1$ is as defined above or is a protected hydroxyl group followed, if appropriate, by removal of the protecting groups; or

(g) reducing an olefin of formula

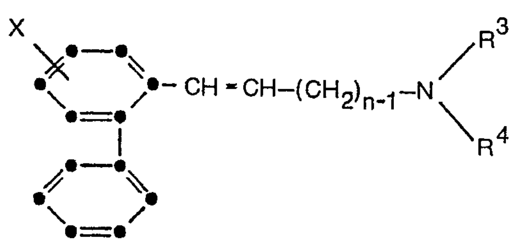

to form a compound of formula (I) in which $R^1$ is hydrogen and Z is $—(CH_2)_n$ where n is 2 to 4.

The biphenylyl alkanolamines provided by this invention, compounds having the above general formula wherein $R^1$ is hydroxy, can be prepared directly by reaction of a biphenyl aldehyde or a biphenyl alkyl ketone with an aminoalkyl Grignard reagent. Such reaction is depicted by the following scheme:

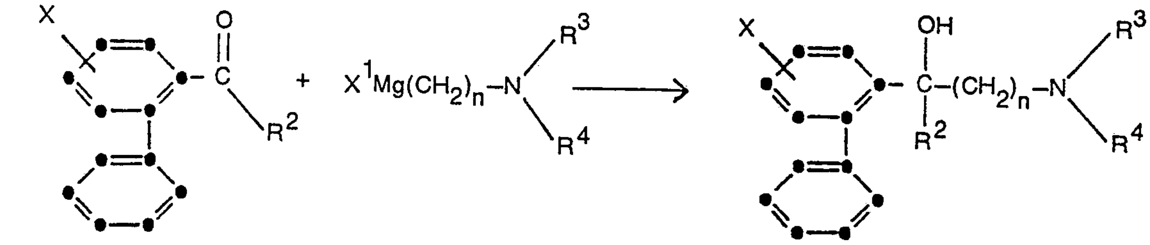

wherein X, $X^1$, $R^2$, $R^3$ and $R^4$ are as defined above and n is 3 or 4. The reaction can be effected by combining approximately equimolar quantities of the biphenyl aldehyde or biphenyl alkyl ketone with an appropriately substituted aminopropyl or aminobutyl magnesium halide in an inert organic solvent, usually an ethereal solvent such as diethyl ether or tetrahydrofuran. When the reaction is carried out at reflux temperature, it normally is substantially complete within four to eight hours. The reaction will normally be effected at a temperature in the range of –25° to 110°C.

An alternative, and preferred process for preparing the biphenylyl butanolamines of this invention, comprises reacting a biphenyl aldehyde or biphenyl alkyl ketone with a suitably N-substituted aminoalkynyl carbanion to form the corresponding biphenylyl alkynylamine, followed by exhaustive reduction to provide the compound of the invention. Such process can be depicted as follows:

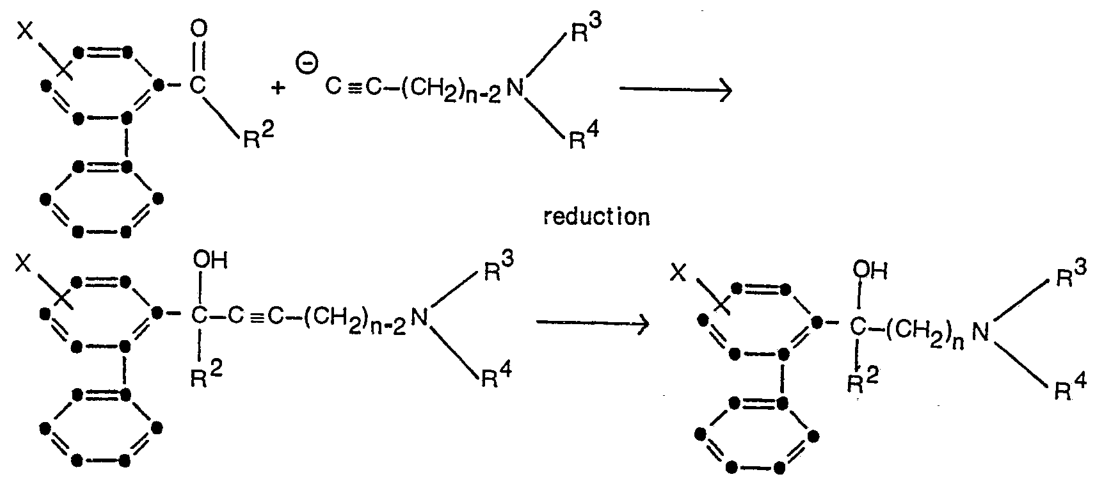


wherein X, $R^2$, $R^3$ and $R^4$ have the above-defined meanings. The process can be carried out by first reacting about equimolar quantities of a biphenyl aldehyde or biphenyl alkyl ketone with an aminoalkynyl carbanion formed by reaction of the corresponding aminoalkyne with a strong base. Strong bases commonly employed to form the propynyl carbanion include methyl lithium, butyl lithium, methyl or ethyl magnesium bromide and triphenylmethyl sodium. The biphenyl aldehyde or ketone generally is mixed with the aminoalkyne and strong base in an unreactive organic solvent such as diethyl ether or tetrahydrofuran, typically at a reduced temperature of about –80° to about –50°C. The alkynyl carbanion is formed *in situ* and then reacts with the aldehyde or ketone. The reaction mixture typically is stirred at a reduced temperature for about one hour, and is then heated to reflux and stirred for an additional eight to ten hours. The intermediate biphenylyl alkynylamine which is formed can be isolated by diluting the reaction mixture with water, thereby decomposing any unreacted carbanion, and then separating the organic layer and removing the solvent therefrom.

The biphenylyl alkynylamine is next reduced to yield the corresponding biphenylyl alkylamine of the invention. Such exhaustive reduction typically is carried out by hydrogenation in the presence of a catalyst such as palladium or platinum. For example, a biphenylyl butynylamine such as N-*iso*pentyl-4-hydroxy-4-(1,1′-biphenyl-2-yl)-2-butynylamine is dissolved in a suitable organic solvent such as methanol or ethanol and stirred for about two to four hours at 20° to 50°C. under a hydrogen atmosphere, in the presence of a hydrogenation catalyst such as palladium suspended on carbon. The reduced product, for instance N-*iso*pentyl-4-hydroxy-4-(1,1′-biphenyl-2-yl)butylamine, is isolated by filtering the reaction mixture to remove the catalyst and then evaporating the reaction solvent from the filtrate. The reduction is preferably effected at a temperature within the range –25° to 110°C. normally at or about room temperature.

The above-described process is a preferred method for preparing the 4-hydroxy-4-(1,1′-biphenyl-2-yl)butylamines of the invention. A preferred method for preparing the 3-hydroxy-3-(1,1′-biphenyl-2-yl)propylamines embraced by the invention comprises reacting a biphenyl alkyl ketone with an amine in the presence of formaldehyde according to Mannich reaction conditions to afford a 3-oxo-3-biphenylyl-propylamine, followed by reduction of the oxo group. For example, a biphenyl methyl ketone such as 2-

acetyl-5-fluoro-1,1′-biphenyl can be reacted with an excess of formaldehyde and an amine of the formula $HNR^3R^4$ in a suitable solvent such as ethanol. The formaldehyde typically is introduced as an aqueous solution of formaldehyde, or as the solid trimer (i.e. trioxymethylene) or as the solid polymer (i.e. polyoxymethylene). The reaction usually is carried out employing a secondary amine such as dimethylamine, methyl ethylamine, diisopropylamine, methyl benzylamine, pyrrolidine or piperidine, thereby avoiding any undesired side reactions which might occur in the presence of a primary amine or ammonia. The reaction normally is conducted at reflux temperature, and routinely is complete within about six to twelve hours. The product, a 3-oxo-3-biphenylylpropylamine of formula (III) can be isolated by evaporation of the reaction solvent, followed by crystallization of the amine, generally as an acid addition salt. The 3-oxo-3-biphenylylpropylamine thus formed next is reduced by reaction with any of a number of chemical reducing agents, including sodium borohydride, or by catalytic hydrogenation using for instance palladium or platinum as catalysts. This reduction will normally be effected at a temperature in the range −25° to 100°C.

The biphenylyl aminoalkyl nitriles of this invention, compounds defined by the above formula (I) wherein $R^1$ is C≡N, can be prepared by alkylating a 1,1′-biphenyl-2-yl-acetonitrile with an aminoalkyl alkylating agent. The reaction can be illustrated by the following reaction schedule:

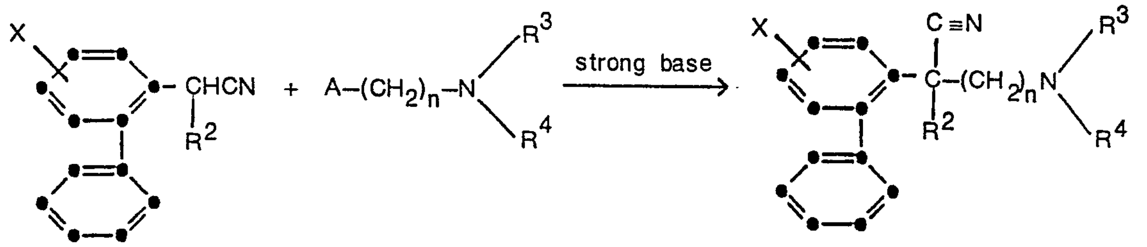


in which X, $R^2$, $R^3$ and $R^4$ are as defined above, n is 2 to 4 and A is a leaving group such as chloro, bromo, iodo, azido, *para*-toluenesulfonyl or methanesulfonyl. The alkylation can be accomplished by first reacting the biphenylyl acetonitrile with a strong base such as sodium or potassium amide, potassium *tert*-butoxide, sodium dimsyl or lithium diisopropylamide in an inert organic solvent such as toluene, benzene, diethyl ether, xylene or tetrahydrofuran. The strong base reacts with the biphenylyl acetonitrile to form a reactive carbanion which, upon addition of the aminoalkyl alkylating agent to the reaction mixture, displaces the leaving group of the alkylating agent and provides a compound of formula (I). The alkylation reaction can be effected at any temperature within the range 30° to 150°C. and for instance typically is complete within eight to sixteen hours when carried out at the reflux temperature of toluene. The product, a 4-cyano-4-biphenylylbutylamine or a 3-cyano-3-biphenylylpropylamine, can be isolated by simply diluting the reaction mixture with water and extracting the product into a suitable solvent such as benzene or diethyl ether.

The biphenylyl aminoalkyl nitriles thus formed are valuable not only as antiarrhythmic agents, but additionally serve as intermediates leading to other compounds of this invention. For example, acid hydrolysis of a biphenylyl aminoalkyl nitrile converts the nitrile moiety to a carboxamide or a carboxylic acid group, depending upon the reaction conditions. For example, reaction of a biphenylyl aminoalkylnitrile such as N,N-di-*n*-butyl-3-cyano-3-(1,1′-biphenyl-2-yl)propylamine with an acid such as ninety percent (v/v) aqueous sulfuric acid for about two to about four hours at a temperature of about 80 to 120°C. converts the cyano group to a carboxamide, thus providing, for instance, N,N-di-*n*-butyl-3-aminocarbonyl-3-(1,1′-biphenyl-2-yl)propylamine. When the reaction is carried out for a longer period of time, for instance for about eight hours or longer, the product is the corresponding carboxylic acid, namely N,N-di-*n*-butyl-3-hydroxycarbonyl-3-(1,1′-biphenyl-2-yl)propylamine. The carboxamide product can be isolated by neutralizing the reaction mixture by the addition of a base such as sodium hydroxide, followed by extraction of the product into a water immiscible solvent such as diethyl ether. Evaporation of the organic solvent then affords the product, generally as a solid which can be crystallized from common solvents such as hexane or Skelly-B. The acids can be isolated by ionexchange chromatography. In both cases the hydrolysis is preferably effected at a temperature within the range from 50° to 150°C. The acids thus formed can be converted into the corresponding $C_{1-4}$ alkyl esters by dissolving them in a $C_{1-4}$ alkanol saturated with HCl and effecting the well-known Fischer-Speier esterification.

The 2-hydroxy-4-biphenylylbutylamines of this invention, i.e. compounds of formula (I) where Z is

$$-CH_2\overset{\displaystyle OH}{\overset{|}{C}H}-CH_2-,$$

can be prepared by reacting an amine of the formula $HNR^3R^4$ with a 4-biphenylyl-1,2-epoxybutane. The epoxybutane can be derived by peracid oxidation of the corresponding 4-biphenylyl-1-butene, which in

turn is prepared by coupling an allyl halide with a biphenylyl methyl halide. The overall reaction is depicted by the following scheme:

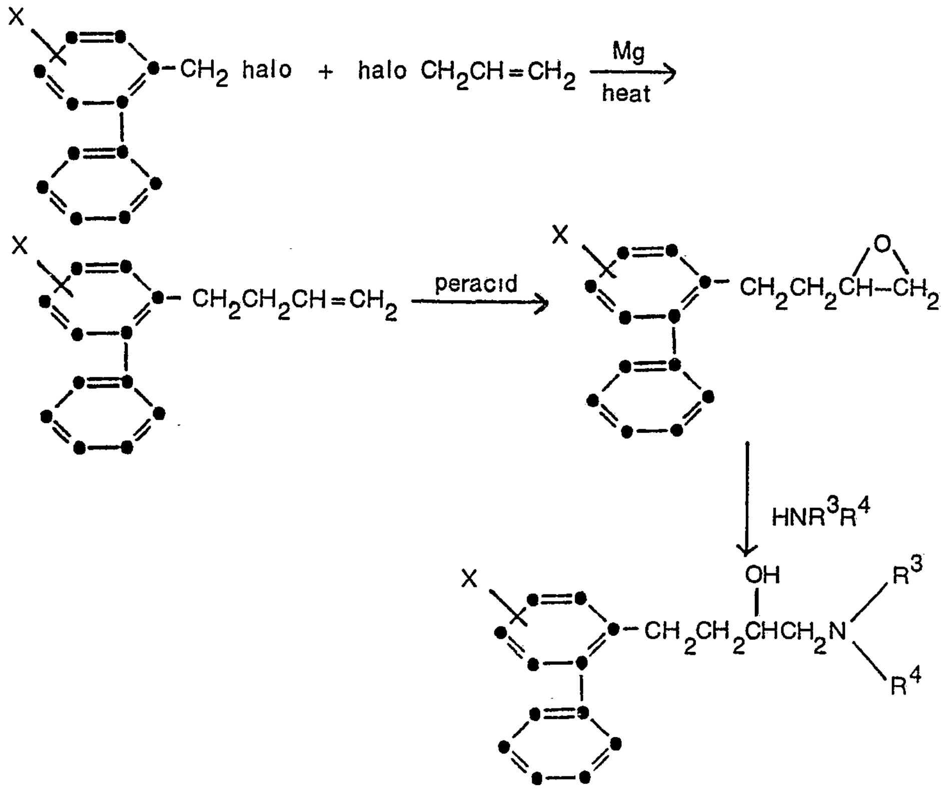


in which X, $R^3$ and $R^4$ are as defined above, and halo is chloro, bromo or iodo.

Compounds of formula (VI) can be derived from starting materials of formula (VII)

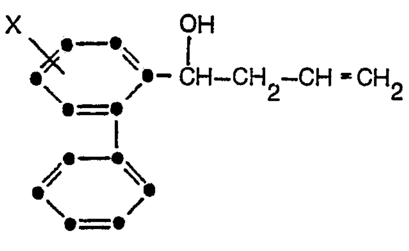

(VII)

The formula (VII) compounds are prepared by the reaction of an aldehyde of formula

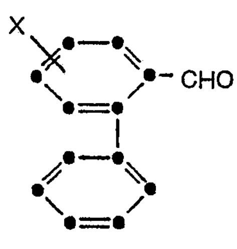

with a Grignard reagent of formula $CH_2{=}CH{-}CH_2{-}MgBr$ (see *Org. Syn., 3,* 121)

The hydroxy compound of formula (VII) can be converted to the corresponding nitrile, amide or carboxylic acid by conventional techniques, e.g. bromination with $PBr_3$, followed by reaction with NaCN to generate the corresponding nitrile and optional hydrolysis to produce the amide or carboxylic acid derivatives. Such compounds may be converted into the corresponding epoxides by treatment with meta-perchlorobenzoic acid. For conversion of the formula (VII) compound to the epoxide it may be necessary to first protect the OH group for example forming the silyl ether (see *Syn. Comm., 6,* 277, (1976)).

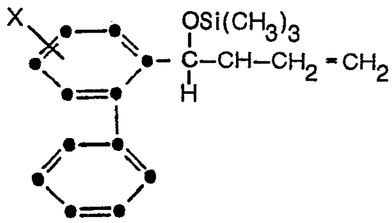

The silyl ether epoxide when formed may be reacted directly with the amine $HNR^3R^4$ to produce the derived compound ($R^1$ is OH) or can first be deprotected.

The condensation of the amine with the 4-biphenylyl-1,2-epoxybutane is generally accomplished by mixing the epoxide with about a two to three molar excess of the amine in an inert organic solvent such as ethanol. The reaction can be carried out in a bomb at a temperature of from 100° to 200°C., and generally at such temperatures is substantially complete within eight to sixteen hours. Less vigorous reaction conditions may also be utilized. The product can be isolated by evaporation of the reaction solvent, and further purification is accomplished by distillation, salt formation or the like.

Biphenylylalkylamines defined by the above general formula wherein $R^1$ is hydrogen and Z is $-(CH_2)_n-$ can be prepared from the corresponding biphenylyl alkanolamines wherein $R^1$ is hydroxy by dehydration to form the corresponding biphenylyl alkenylamine, followed by reduction of the olefinic double bond. For example, a biphenylyl alkanolamine such as N-methyl-N-(2-phenethyl)-3-hydroxy-3-(5-bromo-1,1′-biphenyl-2-yl)propylamine can be dehydrated by reaction with sulfuric acid to provide N-methyl-N-(2-phenethyl)-3-(5-bromo-1,1′-biphenyl-2-yl)-2-propenylamine. Hydrogenation of the latter compound over a suitable catalyst such as palladium on carbon affords the corresponding biphenylyl alkylamine, namely N-methyl-N-(2-phenethyl)-3-(5-bromo-1,1′-biphenyl-2-yl)propylamine.

Compounds of formula (I) in which $R^1$ is $-CONR^5R^6$, where one or both of $R^5$ and $R^6$ represent $C_{1-6}$ alkyl groups, can be prepared by reacting the corresponding acid chloride or ester derivative, i.e. a compound of formula (I) in which $R^1$ is an ester or acid chloride moiety, with excess of a primary or secondary amine of formula $HNR^5R^6$.

The biphenylyl alkylamines comprehended by this invention which bear readily removable groups on the amine nitrogen atom (i.e. $R^3$ or $R^4$ is a readily removable group) are useful both as antiarrhythmic agents and as intermediates. Typical nitrogen substituents which are readily removable include methyl and benzyl. For example, an N-benzyl amine can be de-benzylated by hydrogenation over a catalyst such as palladium on carbon. N-methyl amines can be de-methylated by reaction with a haloformate followed by basic hydrolysis. Any of the above-described processes for preparing compounds of the invention can be carried out so as to provide N-methyl or N-benzyl biphenylyl alkylamines. Such compounds can be de-methylated or de-benzylated to provide compounds of the above general formula wherein one or both of $R^3$ and $R^4$ are hydrogen. Such compounds then can be realkylated as desired to provide any of a number of the other compounds of the invention. For example, a compound such as N-benzyl-N-*iso*propyl-4-cyano-4-(5-chloro-1,1′-biphenyl-2-yl)butylamine can be de-benzylated by hydrogenation to provide the corresponding secondary amine, N-*iso*propyl-4-cyano-4-(5-chloro-1,1′-biphenyl-2-yl)butylamine. The secondary amine then can be realkylated, for instance by reaction with an alkyl halide such as *n*-pentyl bromide or *n*-propyl iodide, an alkenyl halide such as allyl bromide, 3-pentenyl iodide or 2-methyl-2-butenyl bromide, or with a phenylalkyl halide such as 2-phenethyl bromide or 3-phenylpropyl iodide. Such an alkylation process is a convenient method for preparing a wide variety of biphenylyl alkylamines from a common starting material.

As already pointed out, the biphenylyl alkylamines provided by this invention are basic in nature and thus react with acids to form pharmaceutically-acceptable acid addition salts. The salts are prepared by simply mixing the biphenylyl alkylamines with appropriate acids in suitable organic solvents such as diethyl ether. The acid addition salts normally crystallize out of solution and can be collected by filtration and recrystallized if desired.

When the biphenylyl alkylamines comprehended by this invention are tertiary amines, i.e. when $R^3$ and $R^4$ in the above general formula both are other than hydrogen, quaternary ammonium salts are readily formed by reaction with a lower alkyl alkylating agent. For example, reaction of a tertiary amine such as N,N-dimethyl-3-aminocarbonyl-3-(1,1′-biphenyl-2-yl)propylamine with an alkylating agent such as allyl bromide in a suitable solvent such as dichloromethane effects quaternization to afford N-allyl-N,N-dimethyl-3-aminocarbonyl-3-(1,1′-biphenyl-2-yl)propylammonium bromide. Such quaternary salts are characteristically highly crystalline solids, and are included within the scope of this invention as antiarrhythmic agents.

The following list of 1,1′-biphenyl-2-yl propyl and butylamines is illustrative of the compounds comprehended and provided by this invention.

N-*iso*propyl-4-hydroxy-4-(1,1′-biphenyl-2-yl)butylamine;

N-methyl-N-*n*-propyl-4-hydroxy-4-(5-fluoro-1,1′-biphenyl-2-yl)butylamine;

N-*n*-hexyl-4-cyano-4-(1,1′-biphenyl-2-yl)butylamine;

N,N-dimethyl-3-hydroxy-3-(1,1′-biphenyl-2-yl)propylamine;

N-allyl-3-cyano-3-(1,1′-biphenyl-2-yl)propylamine;
N-(3-hexenyl)-N-(2-phenethyl)-4-aminocarbonyl-4-(5-bromo-1,1′-biphenyl-2-yl)butylamine;
N,N-di-*n*-propyl-4-hydroxycarbonyl-4-(1,1′-biphenyl-2-yl)butylamine;
N,N-di*iso*propyl-3-(1,1′-biphenyl-2-yl)propylaminium chloride;
N-*tert*-butyl-4-(5-bromo-1,1′-biphenyl-2-yl)-2-hydroxybutylamine;
N-*iso*pentyl-N-methyl-4-hydroxy-4-methyl-4-(5-chloro-1,1′-biphenyl-2-yl)butylamine;
N,N,N-trimethyl-3-hydroxy-3-(1,1′-biphenyl-2-yl)propylammonium bromide;
d-N,N-diethyl-4-aminocarbonyl-4-(1,1′-biphenyl-2-yl)butylamine;
1-N,N-di-*n*-pentyl-4-hydroxycarbonyl-4-(5-bromo-1,1′-biphenyl-2-yl)butylamine;
d1-N,N-di-*n*-hexyl-4-cyano-4-(1,1′-biphenyl-2-yl)butylamine;
N-(3-pentenyl)-N-*n*-propyl-3-cyano-3-(1,1′-biphenyl-2-yl)propylamine;
N-ethyl-N-methyl-N-*n*-propyl-4-cyano-4-(5-chloro-1,1′-biphenyl-2-yl)butylammonium tosylate;
N-(3-phenylpropyl)-N-allyl-4-hydroxycarbonyl-4-(1,1′-biphenyl-2-yl)butylaminium acetate;
N,N-dimethyl-4-(1,1′-biphenyl-2-yl)-2-hydroxy-butylaminium citrate;
1-[3-cyano-3-(5-fluoro-1,1′-biphenyl-2-yl)propyl]piperidine;
N,N-diethyl-4-hydroxycarbonyl-4-(5-fluoro-1,1′-biphenyl-2-yl)butylamine;
N-benzyl-N-*iso*propyl-3-aminocarbonyl-3-(1,1′-biphenyl-2-yl)propylamine;
1-(3-methylpentyl)-1-ethyl-4-aminocarbonyl-4-(1,1′-biphenyl-2-yl)pyrrolidinium phosphate;
N-allyl-N-(3-phenylpropyl)-N-*n*-propyl-4-methyl-4-(1,1′-biphenyl-2-yl)-2-hydroxybutylammonium iodide; and
N-benzyl-N-(2-phenethyl)-3-cyano-3-(1,1′-biphenyl-2-yl)propylamine.

The biphenylyl alkylamines of this invention are useful as antiarrhythmic agents. That the compounds of the invention are potent antiarrhythmic agents has been demonstrated in dogs suffering from ouabain induced cardiac arrhythmias. In a typical experiment, one or more mongrel dogs of either sex were anesthetized with sodium pentobarbital. A 23 gauge Butterfly infusion needle was placed in the radial vein for the introduction into the dog of sufficient ouabain to induce an arrhythmia, and for the introduction into the dog of the test compound. Each dog was continuously monitored throughout the experiment by electrocardiogram. After the ouabain induced cardiac arrhythmia had continued for thirty minutes, a compound of this invention was administered via the Butterfly infusion needle at the rate of 200 μg per kilogram of dog body weight per minute. If the arrhythmia was not converted to a normal sinus rhythm within ten minutes from the initial administration of test compound, as observed by electrocardiogram, the rate of infusion of test compound was increased to 500 μg per kilogram per minute. When an arrhythmia conversion was noted, the infusion of test compound in general was continued until twice the amount of such compound which effected the conversion was administered to the dog. Following the complete administration of test compound to the dog, the dog's heart was monitored by electrocardiogram until such time that an arrhythmia returned to the dog's heart, or for a maximum time of two hours, at which time the experiment was terminated.

The results of several experiments are set out in the following table. Each compound listed in the table, and its corresponding biological evaluation, is designated by alphabetical letter given in the column marked "compound". Most of the compounds were evaluated more than once, as indicated in the "No. of Dogs" column. The amount of ouabain needed to induce an arrhythmia is given in μg per kilogram of dog body weight. The converting dose and total dose of test compound are given in mg. per kilogram of animal body weight. Duration of conversion is recorded in minutes.

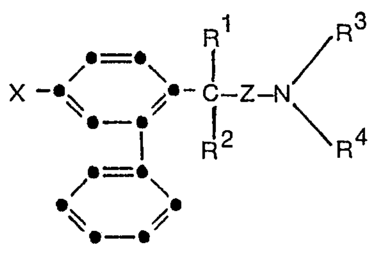

| Compound | X | $R^1$ | $R^2$ | Z | $R^3$ | $R^4$ | No. of Dogs |
|---|---|---|---|---|---|---|---|
| A | F | H | H | $CH_2CH_2CH_2$ | $CH_3$ | $CH_3$ | 2 |
| B | F | OH | H | $CH_2CH_2CH_2$ | Et | Et | 3 |
| C | F | OH | H | $CH_2CH_2CH_2$ | H | iPr | 4 |
| D | Cl | OH | H | $CH_2CH_2CH_2$ | $CH_3$ | $CH_3$ | 3 |
| E | H | OH | H | $CH_2CH_2$ | $CH_3$ | $CH_3$ | 2 |

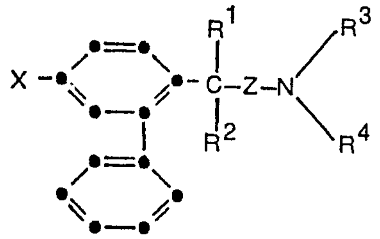

| Compound | X | $R^1$ | $R^2$ | Z | $R^3$ | $R^4$ | No. of Dogs |
|---|---|---|---|---|---|---|---|
| F | H | OH | H | $CH_2CH_2CH_2$ | 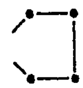 | | 2 |
| G | H | COOH | H | $CH_2CH_2CH_2$ | $CH_3$ | $CH_3$ | 2 |
| H | H | CN | H | $CH_2CH_2CH_2$ | $CH_3$ | $CH_3$ | 1 |
| I | H | H | H | $CH_2\overset{\overset{OH}{\mid}}{C}HCH_2$ | iPr | iPr | 4 |
| J | H | $CONH_2$ | H | $CH_2CH_2CH_2$ | $CH_3$ | $CH_3$ | 2 |

| Compound | Ouabain μg/Kg | Converting dose mg/Kg | Total Dose mg/Kg | Duration of Conversion minutes |
|---|---|---|---|---|
| A | 80 | 4.0 | 8.0 | 12 |
| | 85 | 3.4 | — | — |
| B | 85 | 2.5 | 5.0 | 32 |
| | 75 | 5.8 | 11.5 | 120+ |
| | 75 | 3.5 | 7.0 | 18 |
| C | 60 | 2.8 | 5.5 | 120+ |
| | 70 | 8.0 | 9.5 | 85+ |
| | 80 | 3.0 | 6.0 | 65 |
| | 70 | 3.0 | 6.0 | 105+ |
| D | 70 | 2.5 | 5.0 | 5 |
| | 60 | 0.9 | 1.8 | 120+ |
| | 70 | 6.0 | 10.0 | 43+ |
| E | 70 | 8.5 | 8.7 | 3 |
| | 60 | 1.8 | 3.6 | 51 |
| F | 60 | 2.5 | 5.0 | 93 |
| | 70 | 3.0 | 6.0 | 42 |
| G | 60 | 4.8 | 9.5 | 45 |
| | 75 | 10.0 | 10.0 | — |
| H | 70 | 6.3 | 10.5 | 13 |
| I | 60 | 1.9 | 3.8 | 83 |
| | 55 | 2.0 | 4.0 | 48 |
| | 70 | 1.5 | 3.0 | 26 |
| | 65 | 1.6 | 3.2 | 23 |
| J | 70 | 2.6 | 5.2 | 53 |
| | 60 | 1.9 | 3.8 | 120+ |

The pharmaceutical formulations provided by the invention will normally comprise from about 1 to about 50 per cent by weight of active ingredients. The compounds of this invention can be administered parenterally, for example in the form of liquid injectable solutions or suspensions, or orally in the form of solid compositions which are molded into tablets or encapsulated in gelatin capsules. Pharmaceutical carriers, diluents and excipients commonly used for formulating solid compositions include starch, glucose, lactose, gelatin, malt, rice flour, silica gel, hydroxyethyl cellulose, magnesium carbonate, sodium benzoate and related carriers. Carriers employed in liquid formulations include ethanol, saline, sterile water, glucose syrup, syrup of acacia, peanut oil, wheat germ oil, sorbitan trioleate, ethyl oleate, lecithin and the like. The injectable compositions can be formulated for convenient parenteral administration via the intramuscular, subcutaneous or, preferably, the intravenous routes.

The effective amount of antiarrhythmic agent to be administered may vary depending upon the route of administration, whether or not a subject is suffering from an arrhythmia at the time of administration, the severity of the arrhythmia, the patient being treated and related factors. A subject suffering from an arrhythmia and in need of treatment normally is administered a dose of antiarrhythmic agent sufficient to effect a conversion to normal sinus rhythm. The compound of this invention typically is formulated for parenteral administration and is infused intravenously at a dose effective for causing a conversion of the arrhythmia. The compound then is administered as a maintenance dose, either parenterally or orally. The dose of active compound routinely used to effect a conversion of an arrhythmia is from about 0.1 to about 50 mg. per kg of animal body weight. A typical converting dose administered intravenously is from about 0.5 to about 20 mg. per kg. Maintenance therapy is accomplished by administering an active compound of this invention at the rate of from about 0.1 to about 35 mg. per kg. of animal body weight. A tablet well suited for oral maintenance therapy or prophylactic treatment of a human subject may contain, for example, from about 7 to about 750 mg. of a biphenylyl alkylamine of this invention in combination with a suitable carrier. Such tablet can be administered to such human at the rate of about one to three times each day for the effective control of cardiac arrhythmia.

The following non-limiting examples will further illustrate the invention.

Example 1

N,N-Dimethyl-4-hydroxy-4-(5-fluoro-1,1′-biphenyl-2-yl)butylamine

A solution of 11.6 g. of 2-formyl-5-fluoro-1,1′-biphenyl in 500 ml. of dry tetrahydrofuran containing a Grignard reagent prepared by reacting 9.6 g. of magnesium with 25.3 g. of N,N-dimethyl-3-chloro-propylamine was heated to reflux and stirred for five hours. The reaction mixture was cooled to room temperature, diluted with 200 ml. of saturated aqueous ammonium chloride, and then extracted several times with diethyl ether. The ethereal extracts were combined, washed with water and the solvent was removed by evaporation under reduced pressure to provide an oil. The oil was crystallized from Skelly-B to provide 9.5 g. of N,N-dimethyl-4-hydroxy-4-(5-fluoro-1,1′-biphenyl-2-yl)butylamine. M.P. 60—62°C.

Analysis calc. for $C_{18}H_{22}FNO$

| | | | |
|---|---|---|---|
| Theory: | C, 75.23; | H, 7.72; | N, 4.87. |
| Found: | C, 75.07; | H, 7.68; | N, 4.93. |

Example 2

By a similar procedure, 2-formyl-1,1′-biphenyl was reacted with 3-dimethylaminopropyl magnesium chloride hydrochloride to provide N,N-dimethyl-4-hydroxy-4-(1,1′-biphenyl-2-yl)butylaminium chloride. M.P. 161—164°C.

Analysis calc. for $C_{18}H_{24}ClNO$

| | | | |
|---|---|---|---|
| Theory: | C, 70.69; | H, 7.91; | N, 4.58. |
| Found: | C, 70.47; | H, 7.85; | N, 4.59. |

Example 3

N-*Iso*propyl-4-hydroxy-4-[(1,1′-biphenyl)-2-yl]-2-butynylamine

A solution of 7.8 g. of N-*iso*propyl-2-propynylamine in 500 ml. of dry tetrahydrofuran was stirred and cooled to −80°C. in a dry ice and acetone bath. To the cold stirred reaction mixture was added dropwise over thirty minutes 62.5 ml. of a 1.6 molar solution of *n*-butyl lithium in tetrahydrofuran. Following complete addition, the reaction mixture was stirred at −80°C. for one hour. A solution of 7.3 g. of 2-formylbiphenyl in 500 ml. of tetrahydrofuran next was added dropwise over one hour to the cold stirred reaction mixture. Following complete addition of the aldehyde solution, the reaction mixture was stirred for an additional hour at −80°C. and then heated to reflux and stirred for sixteen hours. After cooling the reaction mixture to room temperature, 500 ml. of water was added slowly to decompose any remaining *n*-butyl lithium. The product next was extracted into diethyl ether, and the ethereal extracts were combined and washed with water. The product then was extracted into 6N

hydrochloric acid solution. The acidic extracts were combined and made alkaline by the addition of 10 percent aqueous sodium hydroxide. The alkaline layer was extracted several times with fresh diethyl ether, and the ethereal extracts were combined, washed with water and dried. Removal of the solvent by evaporation under reduced pressure provided 4.2 g. of N-*iso*propyl-4-hydroxy-4-[(1,1′-biphenyl)-2-yl]-2-butynylamine. M.P. 116—119°C.

Analysis Calc. for $C_{19}H_{21}NO$

| | | | |
|---|---|---|---|
| Theory: | C, 81.68; | H, 7.58; | N, 5.01. |
| Found: | C, 81.78; | H, 7.45; | N, 4.93. |

Example 4—11

The following 4-hydroxy-[(1,1′-biphenyl)-2-yl]-2-butynylamines were prepared from the appropriate propynylamine and formylbiphenyl according to the general procedure set out in Example 3.

N,N-Diethyl-4-hydroxy-4-[(5-fluoro-1,1′-biphenyl)-2-yl]-2-butynylamine. M.P. 133—136°C.

N,N-Di*iso*propyl-4-hydroxy-4-[(5-fluoro-1,1′-biphenyl)-2-yl]-2-butynylamine. M.P. 149—151°C.

N-*iso*propyl-N-methyl-4-hydroxy-4-[(1,1′-biphenyl)-2-yl]-2-butynylamine. $M^+$ 293.

N,N-Di*iso*propyl-4-hydroxy-4-[(1,1′-biphenyl)-2-yl]-2-butynylamine.

N-*tert*-Butyl-4-hydroxy-4-[(1,1′-biphenyl)-2-yl]-2-butynylamine.

N,N-Diethyl-4-hydroxy-4-[(1,1′-biphenyl)-2-yl]-2-butynylamine.

1-(4-hydroxy-4-[(1,1′-biphenyl)-2-yl]-2-butynyl)pyrrolidine.

N-*Iso*propyl-4-hydroxy-4-[(5-fluoro-1,1′-biphenyl)-2-yl]-2-butynylamine.

Example 12

N-*Iso*propyl-4-hydroxy-4-[(5-fluoro-1,1′-biphenyl)-2-yl]butylamine

A solution of 9.0 g. of N-*iso*propyl-4-hydroxy-4-[(5-fluoro-1,1′-biphenyl)-2-yl]-2-butynylamine in 200 ml. of ethanol containing 3.0 g. of five percent palladium suspended on carbon was shaken for two hours at 24°C. in a Parr hydrogenator under an initial hydrogen pressure of 40 psi. The reaction mixture then was filtered and the filtrate was concentrated to an oil by evaporation of the solvent under reduced pressure. The oil was dissolved in 200 ml. of ethyl acetate, and the product was then extracted into 6N hydrochloric acid. The acidic layer was separated, washed with fresh ethyl acetate, and then basified by the addition of 5N sodium hydroxide. The alkaline reaction mixture was extracted several times with diethyl ether, and the ethereal extracts were combined, washed with water and dried. Removal of the solvent by evaporation under reduced pressure afforded a solid which was recrystallized from ethanol to give 1.0 g. of N-*Iso*propyl-4-hydroxy-4-[(5-fluoro-1,1′-biphenyl)-2-yl]butylamine. M.P. 88—91°C.

Analysis calc. for $C_{19}H_{24}FNO$

| | | | |
|---|---|---|---|
| Theory: | C, 75.71; | H, 8.03; | N, 4.65; |
| Found: | C, 75.65; | H, 8.25; | N, 4.64. |

Examples 13—17

According to the general procedure set out in Example 12, the following alkylamines were prepared by hydrogenating the appropriate unsaturated amine:

N,N-Diethyl-4-hydroxy-4-[(5-fluoro-1,1′-biphenyl)-2-yl]butylamine. B.P. 168—174°C. at 0.4 torr.

Analysis calc. for $C_{20}H_{26}FNO$

| | | | |
|---|---|---|---|
| Theory: | C, 76.16; | H, 8.31; | N, 4.44. |
| Found: | C, 75.97; | H, 8.52; | N, 4.67. |

N,N-Diethyl-4-hydroxy-4-[(1,1′-biphenyl)-2-yl]butylamine. B.P. 162—165°C. at 0.4 torr.

Analysis calc. for $C_{20}H_{27}NO$

| | | | |
|---|---|---|---|
| Theory: | C, 80.76; | H, 9.15; | N, 4.71. |
| Found: | C, 80.98; | H, 8.90; | N, 4.84. |

N-*Iso*propyl-N-methyl-4-hydroxy-4-[(1,1′-biphenyl)-2-yl]butylamine. M.P. 50—53°C.

Analysis calc. for $C_{20}H_{27}NO$

| | | | |
|---|---|---|---|
| Theory: | C, 80.76; | H, 9.15; | N, 4.71. |
| Found: | C, 80.55; | H, 9.10; | N, 4.48. |

N-*Iso*propyl-4-hydroxy-4-[(1,1′-biphenyl)-2-yl]butylamine. M.P. 120—121.5°C.

Analysis calc. for $C_{19}H_{25}NO$

| | | | |
|---|---|---|---|
| Theory: | C, 80.52; | H, 8.89; | N, 4.94. |
| Found: | C, 80.51; | H, 8.64; | N, 5.17. |

1-(4-hydroxy-4-[(1,1′-biphenyl)-2-yl]pyrrolidine. M.P. 78—80°C.

Analysis calc. for $C_{20}H_{25}NO$

| | | | |
|---|---|---|---|
| Theory: | C, 81.31; | H, 8.53; | N, 4.74. |
| Found: | C, 81.12; | H, 8.26; | N, 4.86. |

Example 18

N-*tert*-Butyl-4-hydroxy-4-[(5-fluoro-1,1′-biphenyl)-2-yl]butylaminium chloride

To a stirred solution of 5.0 g. of N-*tert*-butyl-4-hydroxy-4-[(5-fluoro-1,1′-biphenyl)-2-yl]butylamine in 200 ml. of diethyl ether was added excess hydrogen chloride. The precipitate which formed was collected by filtration and recrystallized from ethanol and diethyl ether to provide 3.2 g. of N-*tert*-butyl-4-hydroxy-4-[(5-fluoro-1,1′-biphenyl)-2-yl]butylaminium chloride. M.P. 143—145°C.

Analysis calc. for $C_{20}H_{27}ClFNO$

| | | | |
|---|---|---|---|
| Theory: | C, 68.26; | H, 7.73; | N, 3.98. |
| Found: | C, 68.03; | H, 7.98; | N, 3.92. |

Examples 19—23

The following amine acid addition salts were prepared according to the method described in Example 18.

N,N-Di*iso*propyl-4-hydroxy-4-[(5-fluoro-1,1′-biphenyl)-2-yl]butylaminium chloride. M.P. 153—155°C.

Analysis calc. for $C_{22}H_{32}ClFNO$

| | | | |
|---|---|---|---|
| Theory: | C, 69.55; | H, 8.22; | N, 3.69. |
| Found: | C, 69.34; | H, 8.19; | N, 3.61. |

N-*Iso*propyl-4-hydroxy-4-methyl-4-(1,1′-biphenyl-2-yl)butylaminium chloride. M.P. 185—186°C.

Analysis calc. for $C_{20}H_{28}ClNO$

| | | | |
|---|---|---|---|
| Theory: | C, 71.94; | H, 8.45; | N, 4.19. |
| Found: | C, 71.84; | H, 8.48; | N, 4.02. |

N-*Iso*propyl-4-methyl-4-(1,1′-biphenyl-2-yl)butylaminium chloride. M.P. 182—183°C.

Analysis calc. for $C_{20}H_{28}ClNO$

| | | | |
|---|---|---|---|
| Theory: | C, 75.56; | H, 8.88; | N, 4.41. |
| Found: | C, 75.83; | H, 8.57; | N, 4.20. |

N,N-Di*iso*propyl-4-hydroxy-4-(1,1′-biphenyl-2-yl)butylaminium chloride. M.P. 172—174°C.

Analysis calc. for $C_{22}H_{32}ClNO$

| | | | |
|---|---|---|---|
| Theory: | C, 73.00; | H, 8.91; | N, 3.87. |
| Found: | C, 72.80; | H, 8.61; | N, 3.93. |

N-*Iso*propyl-N-methyl-4-hydroxy-4-(1,1′-biphenyl-2-yl)butylaminium chloride. M.P. 130—133°C.

Analysis calc. for $C_{20}H_{28}ClNO$

| | | | |
|---|---|---|---|
| Theory: | C, 71.94; | H, 8.45; | N, 4.19. |
| Found: | C, 72.08; | H, 8.22; | N, 3.92. |

Example 24

N,N-Dimethyl-3-hydroxy-3-(1,1′-biphenyl-2-yl)propylamine

A solution of 10.1 g. of 2-acetyl-1,1′-biphenyl in 100 ml. of ethanol containing 7.6 g. of paraformaldehyde, 9.7 g. of dimethylaminium chloride and one drop of concentrated hydrochloric acid was heated to reflux and stirred for sixteen hours. After cooling the reaction mixture to room temperature, the solvent was removed by evaporation. The residue thus formed was crystallized from ethanol and diethyl ether to give 3.6 g. of N,N-dimethyl-3-oxo-3-(1,1′-biphenyl-2-yl)propylaminium chloride. M.P. 130—131°C.

To a cold (0°C.) stirred solution of N,N-dimethyl-3-oxo-3-(1,1′-biphenyl-2-yl)propylamine in 300 ml. of methanol was added portion-wise over thirty minutes 1.9 g. of sodium borohydride. Following complete addition of the reducing agent, the reaction mixture was warmed to room temperature and stirred for sixteen hours, and then heated to reflux and stirred for an additional three hours. The

reaction mixture was cooled to 25°C. and concentrated to dryness by evaporation of the solvent under reduced pressure, thus providing a solid. The solid so formed was recrystallized from Skelly B solvent to afford 1.5 g. of N,N-dimethyl-3-hydroxy-3-(1,1'-biphenyl-2-yl)propylamine. M.P. 80—82°C.

Analysis Calc. for $C_{17}H_{21}NO$

| | | | |
|---|---|---|---|
| Theory: | C, 79.96; | H, 8.29; | N, 5.49. |
| Found: | C, 80.25; | H, 8.49; | N, 5.22. |

Example 25

N,N-Dimethyl-4-cyano-4-(1,1'-biphenyl-2-yl)butylamine

To a stirred solution of 3.5 g. of sodium amide in 250 ml. of dry toluene was added dropwise over thirty minutes a solution of 16.7 g. of (1,1'-biphenyl-2-yl)acetonitrile in 200 ml. of toluene. The reaction mixture was heated to reflux and stirred for three hours following complete addition of the nitrile. The reaction mixture next was cooled to room temperature, and to the stirred solution was added dropwise over one hour a solution of 17.4 g. of N,N-dimethyl-3-chloropropylamine in 200 ml. of dry toluene. When the addition was complete, the reaction mixture was heated to reflux and stirred for sixteen hours. After cooling the reaction mixture to room temperature, it was added to 500 ml. of ice water. The product was extracted from the aqueous mixture into diethyl ether, and the ethereal extracts were combined, washed with water and then extracted several times with 6N hydrochloric acid. The acidic extracts were combined, washed with fresh diethyl ether and then made alkaline with 10% sodium hydroxide. The alkaline solution was extracted with fresh diethyl ether, and the ethereal extracts were combined, washed with water and dried. Removal of the solvent by evaporation under reduced pressure and distillation of the product provided 11.3 g. of N,N-dimethyl-4-cyano-4-(1,1'-biphenyl-2-yl)butylamine. B.P. 157—162°C. at 0.5 torr.

Analysis calc. for $C_{19}H_{22}N_2$

| | | | |
|---|---|---|---|
| Theory: | C, 81.97; | H, 7.97; | N, 10.06. |
| Found | C, 81.74; | H, 7.71; | N, 9.89. |

Examples 26—27

Following the procedure set forth in Example 25, the following cyano-aralkyl amines were prepared.

N,N-Di*iso*propyl-4-cyano-4-(1,1'-biphenyl-2-yl)butylamine. B.P. 75—185°C. at 0.3 torr. $M^+$ 334.

N-Benzyl-N-*iso*propyl-4-cyano-4-(1,1'-biphenyl-2-yl)butylamine. B.P. 195—204°C. at 0.1 torr. $M^+$ 382.

Example 28

N,N-Dimethyl-4-aminocarbonyl-4-(1,1'-biphenyl-2-yl)butylamine

A solution of 2.4 g. of N,N-dimethyl-4-cyano-4-(1,1'-biphenyl-2-yl)butylamine in 20 ml. of concentrated sulfuric acid containing 5 ml. of water was heated on a steam bath for four hours. The reaction mixture then was cooled to 5°C. and made alkaline by the addition of 10% sodium hdyroxide. The aqueous alkaline solution was extracted several times with diethyl ether. The ethereal extracts were combined, washed with water and dried. Removal of the solvent by evaporation under reduced pressure afforded a solid, which upon recrystallization from benzene and Skelly-B solvent provided 600 mg. of N,N-dimethyl-4-aminocarbonyl-4-(1,1'-biphenyl-2-yl)butylamine. M.P. 108—110°C.

Analysis calc. for $C_{19}H_{24}N_2O$

| | | | |
|---|---|---|---|
| Theory: | C, 76.99; | H, 8.16; | N, 9.45. |
| Found: | C, 76.70; | H, 7.94; | N, 9.24. |

Examples 29—30

Following the procedure of Example 28, the following amides were prepared by acid hydrolysis of the corresponding nitriles:

N,N-Diisopropyl-4-aminocarbonyl-4-(1,1'-biphenyl-2-yl)butylamine. M.P. 70—72°C.

Analysis calc. for $C_{23}H_{32}N_2O$

| | | | |
|---|---|---|---|
| Theory: | C, 78.36; | H, 9.15; | N, 7.95. |
| Found: | C, 78.08; | H, 8.89; | N, 7.71. |

N-Benzyl-N-*iso*propyl-3-aminocarbonyl-3-(1,1'-biphenyl-2-yl)butylamine.

Example 31

N,N-Dimethyl-4-hydroxycarbonyl-4-(1,1'-biphenyl-2-yl)butylamine

A solution of 6.0 g. of N,N-dimethyl-4-cyano-4-(1,1'-biphenyl-2-yl)butylamine in 65 ml. of 90% sulfuric acid was heated on a steam bath for eight hours. After cooling the reaction mixture to 5°C. and making it alkaline by the addition of 10% sodium hydroxide, the product was extracted therefrom into

diethyl ether. The ethereal extracts were washed with water and dried. Excess hydrogen chloride was bubbled into the ethereal solution, whereupon a precipitate formed and was collected by filtration. Crystallization of the precipitate from ethanol afforded 2.9 g. of N,N-dimethyl-4-hydroxycarbonyl-4-(1,1'-biphenyl-2-yl)butylaminium chloride. M.P. 192—194°C.

Analysis calc. for $C_{19}H_{24}ClNO_2$

| | | | | |
|---|---|---|---|---|
| Theory: | C, 68.35; | H, 7.25; | N, 4.20; | O, 9.58. |
| Found: | C, 68.18; | H, 7.19; | N, 4.39; | O, 10.00. |

### Example 32

N,N-Di*iso*propyl-2-hydroxy-4-(1,1'-biphenyl-2-yl)butylamine

A solution of 4.0 g. of 1,2-epoxy-4-(1,1'-biphenyl-2-yl)butane in 50 ml. of ethanol containing 6.0 g. of di*iso*propylamine was heated to 160°C. in a reaction bomb and stirred at that temperature for sixteen hours. The reaction mixture was then cooled to room temperature and concentrated to an oil by evaporation of the solvent under reduced temperature. The oil was dissolved in 200 ml. of diethyl ether, washed with water and then extracted into 6N hydrochloric acid. The acid layer was separated, washed with fresh diethyl ether, and then made alkaline by the addition of 10% sodium hydroxide. The alkaline solution was extracted several times with diethyl ether. The ethereal extracts were combined, washed with water and dried. Removal of the solvent by evaporation provided an oil, which upon distillation afforded 1.8 g. of N,N-Di*iso*propyl-2-hydroxy-4-(1,1'-biphenyl-2-yl)butylamine. B.P. 168—171°C. at 0.2 torr.

Analysis calc. for $C_{22}H_{31}NO$

| | | | |
|---|---|---|---|
| Theory: | C, 81.18; | H, 9.60; | N, 4.30. |
| Found: | C, 81.42; | H, 9.82; | N, 4.26. |

### Example 33

Following a precedure similar to that of Example 32, 1,2-epoxy-4-(1,1'-biphenyl-2-yl)butane was reacted with *iso*propylamine to provide N-*iso*propyl-2-hydroxy-4-(1,1'-biphenyl-2-yl)butylamine. The amine base so formed was reacted with hydrogen chloride to provide N-*iso*propyl-2-hydroxy-4-(1,1'-biphenyl-2-yl)butylaminium chloride. M.P. 153—154°C.

Analysis calc. for $C_{19}H_{25}ClNO$

| | | | |
|---|---|---|---|
| Theory: | C, 71.34; | H, 8.19; | N, 4.38. |
| Found: | C, 71.21; | H, 7.95; | N, 4.36. |

### Example 34

N,N-Dimethyl-4-(1,1'-biphenyl-2-yl)butylamine

A solution of 4.6 g. of N,N-dimethyl-4-hydroxy-4-(1,1'-biphenyl-2-yl)butylaminium chloride in 100 ml. of 4N sulfuric acid was heated to 100°C. and stirred for one hour. The reaction mixture was cooled to 5°C. and made alkaline by the addition of 10% sodium hydroxide. The alkaline solution was extracted with diethyl ether, and the ethereal extracts were combined, washed with water and dried. Evaporation of the solvent under reduced pressure afforded 3.3 g. of N,N-dimethyl-4-(1,1'-biphenyl-2-yl)-3-butenylamine.

A solution of 3.0 g. of N,N-dimethyl-4-(1,1'-biphenyl-2-yl)-3-butenylamine in 200 ml. of ethyl acetate containing 2.0 g. of five percent palladium on carbon was stirred for one hour at 25°C. under hydrogen at 40 psi. The reaction mixture then was filtered and the solvent was removed from the filtrate by evaporation to provide an oil. The oil was dissolved in 100 ml. of diethyl ether and stirred while excess hydrogen chloride was added to the solution. The precipitate which formed was collected by filtration and was recrystallized from ethanol and diethyl ether to provide 2.1 g. of N,N-dimethyl-4-(1,1'-biphenyl-2-yl)butylaminium chloride. M.P. 114—116°C.

Analysis calc. for $C_{18}H_{24}ClNO$

| | | | |
|---|---|---|---|
| Theory: | C, 74.59; | H, 8.35; | N, 4.83. |
| Found: | C, 74.48; | H, 8.30; | N, 4.71. |

### Example 35

N-*Iso*propyl-3-aminocarbonyl-3-(1,1'-biphenyl-2-yl)propylamine

A solution of 2.1 g. of N-benzyl-N-*iso*propyl-3-aminocarbonyl-3-(1,1'-biphenyl-2-yl)propylamine (from Example 30) in 200 ml. of ethanol containing 2.0 g. of five percent palladium suspended on carbon was stirred under a hydrogen pressure of 60 psi and heated to 40°C. for forty-five minutes. The reaction mixture then was filtered and the filtrate was concentrated in volume by evaporation of the solvent. The residue thus obtained was dissolved in diethyl ether, washed with water and then crystallized from Skelly-B to afford 150 mg. of N-*iso*propyl-3-aminocarbonyl-3-(1,1'-biphenyl-2-yl)propylamine. M.P. 92—95°C.

Analysis calc. for $C_{19}H_{24}N_2O$

| | | | |
|---|---|---|---|
| Theory: | C, 76.99; | H, 8.16; | N, 9.45. |
| Found: | C, 76.78; | H, 8.14; | N, 9.57. |

Example 36

Following a similar procedure, the appropriate N-benzyl amine was hydrogenated to give the following compound:

N-*iso*propyl-4-aminocarbonyl-4-(1,1′-biphenyl-2-yl)butylamine. M.P. 121—122°C.

Analysic calc. for $C_{20}H_{26}N_{20}$

| | | | |
|---|---|---|---|
| Theory: | C, 77.38; | H, 8.44; | N, 9.02. |
| Found: | C, 77.19; | H, 8.16; | N, 8.91. |

Example 37

1-methyl-1-[4-hydroxy-4-(1,1′-biphenyl-2-yl)butyl]pyrrolidinium iodide

To a stirred solution of 3.0 g. of 1-[4-hydroxy-4-(1,1′-biphenyl-2-yl)butyl]pyrrolidine in 10 ml. of ethanol was added 1.3 g. of methyl iodide in one portion. The reaction mixture was stirred at room temperature for sixteen hours, and the solvent was then removed by evaporation under reduced pressure to provide an oil. The oil was crystallized from petroleum ether to provide 4.2 g. of 1-methyl-1-[4-hydroxy-4-(1,1′-biphenyl-2-yl)butyl]pyrrolidinium iodide. M.P. 157—159°C.

Analysis calc. for $C_{21}H_{28}INO$

| | | | |
|---|---|---|---|
| Theory: | C, 57.67; | H, 6.45; | N, 3.20. |
| Found: | C, 57.75; | H, 6.19; | N, 3.22. |

Example 38

The following quaternary ammonium salt was prepared by reacting the appropriate tertiary amine with an alkylating agent according to the method described in Example 37.

N,N-Dimethyl-N-*iso*propyl-4-hydroxy-4-(1,1′-biphenyl-2-yl)butylammonium iodide. M.P. 93—95°C.

Analysis calc. for $C_{21}H_{30}INO$

| | | | |
|---|---|---|---|
| Theory: | C, 57.41; | H, 6.88; | N, 3.19. |
| Found: | C, 56.93; | H, 6.69; | N, 3.19. |

Example 39

N,N-Dimethyl-4-hydroxy-4-[(5-chloro-1,1′-biphenyl)-2-yl]butylaminium chloride

Magnesium (5.0 g., 0.21 mole) was added to a 3-litre, 3-necked flask equipped with a stirrer and thermometer. Sufficient ether was added just to cover the magnesium and this was followed by the slow addition of a little ethylene dibromide to activate the mixture. N,N-dimethyl-3-chloropropylamine (31.6 g., 0.2 mole) dissolved in diethyl ether was then added to the reaction mixture which was stirred at room temperature until substantially all of the magnesium had dissolved. 2-Phenyl-4-chloro-benzaldehyde (48.7 g., 0.23 mole) dissolved in diethylether was then added dropwise and the reaction mixture stirred overnight. Next morning, the reaction mixture was refluxed gently for about three hours. This was followed by decomposition with saturated ammonium chloride and addition to diethyl ether, extraction with 6N HCl, basification with 10% NaOH and repeat extraction with diethyl ether, washing with water and drying with sodium sulphate. After concentration a solid crystallized out and this was dissolved in diethyl ether and converted to the title product with HCl, m.p. 119—121°C., yield 13.7 g.

Example 40

N,N-Dimethyl-4-[(5-chloro-1,1′-biphenyl)-2-yl]-3-butenylaminium chloride

The product of Example 39 (3.0 g., 0.1 mole) was refluxed for six hours in 4N $H_2SO_4$ (80 ml.) in a 250 ml. flask with stirring. The reaction mixture was then cooled and added to ice water, basified with 10% NaOH, extracted with diethyl ether, washed with water, dried with $Na_2SO_4$ and concentrated down. The base thereby formed was converted into a hydrochloride (the title compound) and then recrystallized from ethanol and diethyl ether, m.p. 149.5° to 150°C., yield 1.6 g.

Example 41

N,N-Dimethyl-4-[(5-chloro-1,1′-biphenyl)-2-yl]butylaminium chloride

N,N-Dimethyl-4-[(5-chloro-1,1′-biphenyl)-2-yl]-3-butenylaminium chloride (8.4 g., 0.011 mole) was hydrogenated in the presence of 5% palladium on charcoal (1.0 g.) in ethanol (200 ml.) using a procedure similar to that of Example 34, m.p. 131°—133°C., yield 1.8 g.

Example 42

N-*iso*Propyl-5-aminocarbonyl-5-(1,1′-biphenyl-2-yl)pentylamine, hydrochloride

(a) 5-Cyano-5-(1,1′-biphenyl-2-yl)pentyl chloride

1,1′-Biphenyl-2-yl acetonitrile (19.3 g., 0.1 mole) was heated with 1-bromo-4-chlorobutane (18.1 g., 0.11 mole) in the presence of sodamide (4.3 g., 0.11 mole) under a nitrogen atmosphere using dry toluene (500 ml) as a solvent to yield the title compound, b.p. 141—65°C/0.6 mm., yield 16.5 g.

(b) N-*iso*Propyl-5-cyano-5-(1,1′-biphenyl-2-yl)pentylamine, hydrochloride

5-Cyano-5-(1,1′-biphenyl-2-yl)pentyl chloride (13.7 g.) was reacted with *iso*propylamine (100 ml.) in a bomb at 150°C. in the presence of potassium iodide (2 g.) overnight. The amine base thus formed was converted into the hydrochloride salt, m.p. 143—5°C. (recrystallized from EtOH/$Et_2O$), yield 4.9 g.

(c) The nitrile prepared in (b) above (4.9 g.) was heated with 90% $H_2SO_4$ (20.0 g.) to form the aminocarbonyl derivative. This material was then converted to the hydrochloride, i.e. N-*iso*propyl-5-aminocarbonyl-5-(1,1′-biphenyl-2-yl)pentylamine, hydrochloride, m.p. 215—217°C. (EtOH/$Et_2O$) yield 3.7 g.

Example 43

Formulation suitable for oral administration.

| Ingredient | Mg. |
|---|---|
| N-*iso*propyl-4-hydroxy-4-(1,1′-biphenyl-2-yl)butylaminium chloride | 25 |
| starch | 200 |
| sucrose | 275 |
| | 500 |

The above ingredients were thoroughly mixed with a suitable lubricant and the mixture molded into a tablet.

Example 44

Formulation suitable for intravenous administration.

| Ingredient | |
|---|---|
| N,N-diethyl-4-aminocarbonyl-4-(5-fluoro-1,1′-biphenyl-2-yl)butylaminium chloride | 250 mg. |
| isotonic saline | 500 ml. |
| 10%aqueous glucose | 500 ml. |

The above ingredients were mixed together to form an infusion solution.

**Claims**

1. A compound of formula (I):

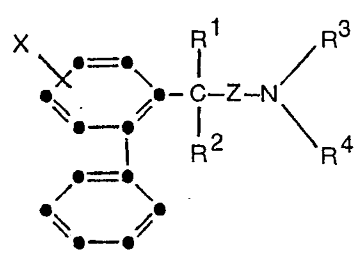

(I)

wherein:

X is hydrogen or a halo substituent at any position in the phenyl ring;

$R^1$ is hydrogen, hydroxy, C≡N, $CONR^5R^6$, where $R^5$ and $R^6$ independently represent hydrogen or $C_{1-6}$ alkyl, or $CO_2R^8$ where $R^8$ is hydrogen or $C_{1-4}$ alkyl;

$R^2$ is hydrogen or $C_{1-3}$ alkyl;

$$Z \text{ is } -(CH_2)_n- \text{ or } -CH_2\overset{OH}{\overset{|}{C}}HCH_2-;$$

where n is an integer of from 2 to 4;

$R^3$ and $R^4$ independently are hydrogen, $C_{1-6}$ alkyl, $CH_2C_{2-5}$ alkenyl, phenyl $C_{1-3}$ alkyl, or taken together with the adjacent nitrogen atom form a heterocyclic group containing from 5 to 7 ring atoms and optionally containing one heteroatom selected from oxygen and nitrogen;

or a pharmaceuticall-acceptable salt thereof.

2. A compound of formula (I) as claimed in claim 1, wherein X is hydrogen, or fluoro, chloro or bromo situated at the 5-position of the biphenylyl ring; $R^1$ is hydrogen, hydroxy, C≡N, $CONH_2$ or COOH; $R^2$ is hydrogen or methyl, Z is $-(CH_2)_n-$ where n is 2 or 3 or is $-CH_2CH(OH)CH_2-$ when $R^1$ is hydrogen; and $R^3$ and $R^4$ independently are hydrogen, $C_{1-6}$ alkyl, $CH_2C_{2-5}$ alkenyl, phenyl $C_{1-3}$ alkyl, or taken together with the adjacent nitrogen atom form a heterocyclic group selected from pyrrolidino, piperidino and morpholino; or a pharmaceutically-acceptable acid addition or quaternary ammonium salt thereof.

3. A compound of formula (I) as claimed in claim 1 or 2, wherein $R^1$ is hydrogen.

4. A compound of formula (I) as claimed in any one of claims 1 to 3, wherein $R^1$ is $CONH_2$.

5. A compound of formula (I) as claimed in any one of the preceding claims, wherein $R^3$ is hydrogen and $R^4$ is *iso*-propyl.

6. A compound of formula (I) as claimed in any one of the preceding claims, wherein Z is $-(CH_2)_n-$ where n is 3.

7. N-*Iso*propyl-4-aminocarbonyl-4-(1,1'-biphenyl-2-yl)butylamine, or a pharmaceutically-acceptable salt thereof.

8. A process for preparing a compound of formula (I) as claimed in any one of claims 1 to 7 which comprises:

(a) reacting a ketone of formula (II)

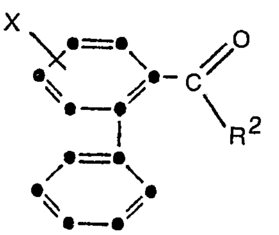

(II)

with a Grignard reagent of formula:

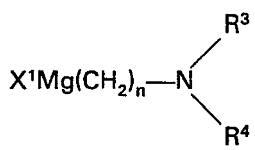

where $X^1$ is chloro, bromo or iodo and n is 3 or 4, to produce a compound of formula (I) in which $R^1$ is hydroxy and Z is $-(CH_2)_n-$ where n is 2 to 4;

(b) reducing a compound of formula (II)

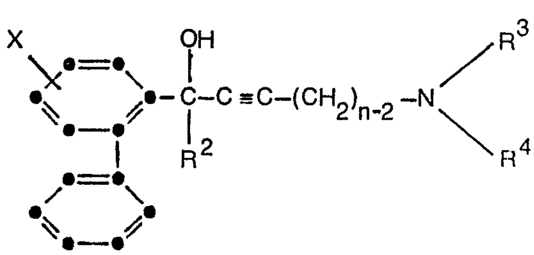

(II)

to form a compound of formula (I) where $R^1$ is hydroxy and Z is $-(CH_2)_n-$ where n is 3 or 4;

(c) reducing a ketone of formula (III)

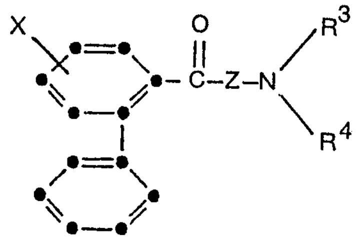

(III)

to form a compound of formula (I) in which $R^1$ is hydroxy and $R^2$ is hydrogen;

(d) alkylating a nitrile of formula (IV);

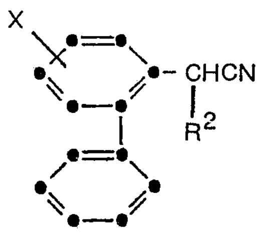

(IV)

with an aminoalkylating agent of formula:

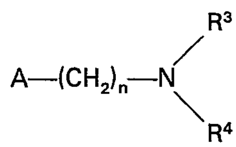

where A is a leaving group; to form a compound of formula (I) where $R^1$ is —C≡N;

(e) hydrolyzing a nitrile of formula (V):

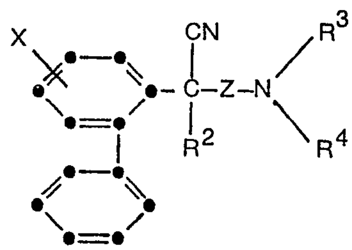

(V)

to form a compound of formula (I) in which $R^1$ is $-CONH_2$ or —COOH;

(f) condensing an epoxide of formula (VI)

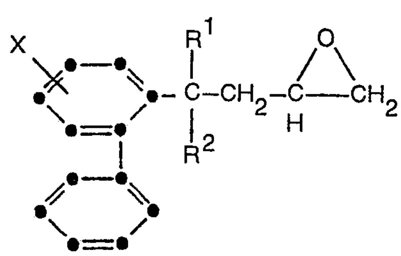

(VI)

with an amine of formula $HNR^3R^4$, where $R^1$ is as defined above or is a protected hydroxyl group followed, if appropriate, by removal of the protecting groups; or

(g) reducing an olefin of formula

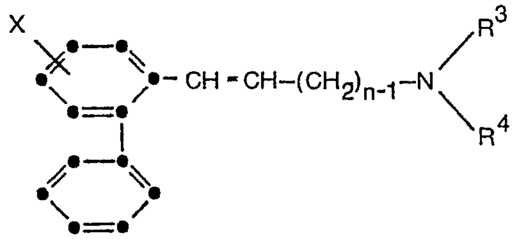

to form a compound of formula (I) in which $R^1$ is hydrogen and Z is $-(CH_2)_n$ where n is 2 to 4.

9. A pharmaceutically-acceptable formulation which comprises as an active ingredient a compound of formula (I), or a pharmaceutically-acceptable salt thereof, as claimed in any one of claims 1 to 7, associated with a pharmaceutically-acceptable carrier therefor.

10. A compound of formula (I), or a pharmaceutically-acceptable salt thereof, as claimed in any one of claims 1 to 7, for use in cardiovascular disorders.

## Revendications

1. Composé de formule (I):

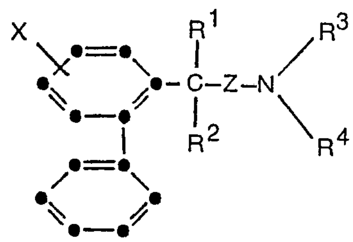

(I)

dans laquelle:

X représente un atome d'hydrogène ou un substituant halo en n'importe quelle position du noyau phényle;

$R^1$ représente un atome d'hydrogène, un groupe hydroxy, un groupe C≡N, un groupe $CONR^5R^6$ où $R^5$ et $R^6$ représentent indépendamment l'un de l'autre un atome d'hydrogène ou un groupe alkyle en $C_1$—$C_6$, ou un groupe $CO_2R^8$ où $R^8$ représente un atome d'hydrogène ou un groupe alkyle en $C_1$—$C_4$;

$R^2$ représente un atome d'hydrogène ou un groupe alkyle en $C_1$—$C_3$;

Z représente un groupe $-(CH_2)_n-$ ou $-CH_2CH(OH)CH_2-$;

où n est un nombre entier de 2 à 4;

$R^3$ et $R^4$ représentent indépendamment l'un de l'autre un atome d'hydrogène, un groupe alkyle en $C_1$—$C_6$, un groupe $CH_2$-alcényle en $C_2$—$C_5$, un groupe phényl-alkyle en $C_1$—$C_3$ ou, pris ensemble avec l'atome d'azote adjacent, ils forment un groupe hétérocyclique contenant 5 à 7 atomes cycliques et éventuellement un hétéro-atome choisi parmi l'oxygène et l'azote;

ou un sel pharmaceutiquement acceptable de ce composé.

2. Composé de formule (I) suivant la revendication 1, formule dans laquelle X représente un atome d'hydrogène, de fluor, de chlore ou de brome occupant la position 5 du noyau biphénylyle; $R^1$ représente un atome d'hydrogène, un groupe hydroxy, C≡N, $CONH_2$ ou COOH; $R^2$ représente un atome d'hydrogène ou un groupe méthyle, Z représente un groupe $-(CH_2)_n-$ où n est égal à 2 ou 3, ou un groupe $-CH_2CH(OH)CH_2-$ lorsque $R^1$ est un atome d'hydrogène; et $R^3$ et $R^4$ représentent indépendamment l'un de l'autre un atome d'hydrogène, un groupe alkyle en $C_1$—$C_6$, un groupe $CH_2$-alcényle en $C_2$—$C_5$, un groupe phényl-alkyle en $C_1$—$C_3$ ou, pris ensemble avec l'atome d'azote adjacent, ils forment un groupe hétérocyclique choisi parmi les groupes pyrrolidino, pipéridino et morpholino; ou un sel d'ammonium quaternaire ou d'addition d'acide pharmaceutiquement acceptable de ce composé.

3. Composé de formule (I) suivant la revendication 1 ou 2, formule dans laquelle $R^1$ est un atome d'hydrogène.

4. Composé de formule (I) suivant l'une quelconque des revendications 1 à 3, formule dans laquelle $R^1$ est un groupe $CONH_2$.

5. Composé de formule (I) suivant l'une quelconque des revendications précédentes, formule dans laquelle $R^3$ est un atome d'hydrogène et $R^4$ est un groupe *iso*propyle.

6. Composé de formule (I) suivant l'une quelconque des revendications précédentes, formule dans laquelle Z est un groupe $-(CH_2)_n-$ où n est égal à 3.

7. La N-*iso*propyl-4-aminocarbonyl-4-(1,1'-biphényl-2-yl)butylamine ou un de ses sels pharmaceutiquement acceptables.

8. Procédé de préparation d'un composé de formule (I) suivant l'une quelconque des revendications 1 à 7, caractérisé en ce qu'il comprend les étapes qui consistent à:

(a) faire réagir une cétone de formule (II)

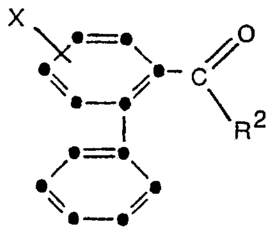

(II)

avec un réactif de Grignard de formule:

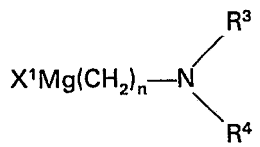

dans laquelle $X^1$ représente un atome de chlore, de brome ou d'iode et n est égal à 3 ou 4, pour obtenir un composé de formule (I) dans laquelle $R^1$ est un groupe hydroxy et Z est un groupe $-(CH_2)_n-$ où n est un nombre de 2 à 4;

(b) réduire un composé de formule (II):

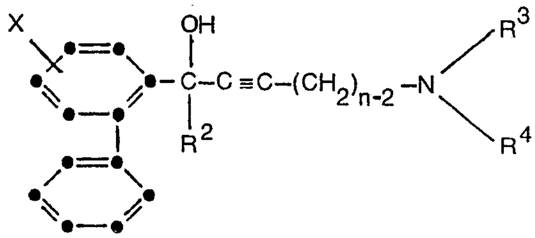

(II)

pour former un composé de formule (I) dans laquelle $R^1$ est un groupe hydroxy et Z est un groupe $-(CH_2)_n-$ où n est égal à 3 ou 4;

(c) réduire une cétone de formule (III):

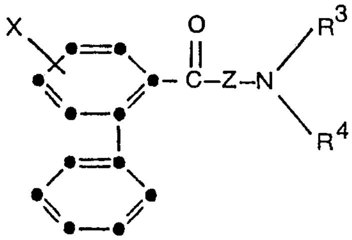

(III)

pour former un composé de formule (I) dans laquelle $R^1$ est un groupe hydroxy et $R^2$ est un atome d'hydrogène;

(d) alkyler un nitrile de formule (IV):

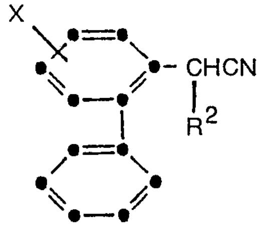

(IV)

avec un agent d'aminoalkylation de formule:

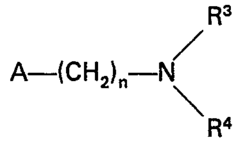

dans laquelle A est un groupe qui s'éloigne; pour former un composé de formule (I) dans laquelle $R^1$ est un groupe —C≡N;

(e) hydrolyser un nitrile de formule (V):

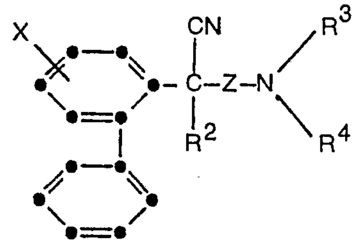

(V)

pour former un composé de formule (I) dans laquelle $R^1$ est un groupe $—CONH_2$ ou —COOH;

(f) condenser un époxyde de formule (VI):

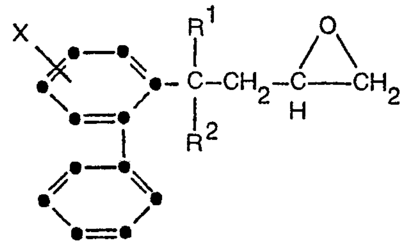

(VI)

avec une amine de formule $HNR^3R^4$ dans laquelle $R^1$ a la signification définie ci-dessus ou est un groupe hydroxy protégé, cette condensation étant suivie, si cela s'avère approprié, de l'élimination des groupes protecteurs; ou

(g) réduire une oléfine de formule:

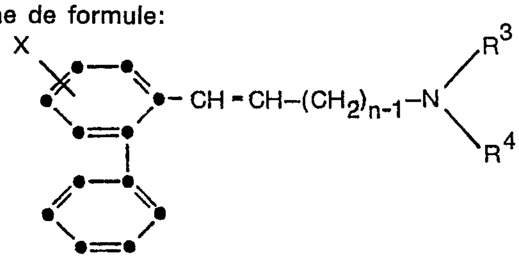

pour former un composé de formule (I) dans laquelle $R^1$ est un atome d'hydrogène et Z est un groupe $—(CH_2)_n$ où n est un nombre de 2 à 4.

9. Formulation pharmaceutiquement acceptable comprenant, comme ingrédient actif, un composé de formule (I) ou un de ses sels pharmaceutiquement acceptables suivant l'une quelconque des revendications 1 à 7, en association avec un support pharmaceutiquement acceptable.

10. Composé de formule (I) ou un de ses sels pharmaceutiquement acceptables suivant l'une quelconque des revendications 1 à 7, en vue de l'utiliser pour les troubles cardiovasculaires.

**Patentansprüche**

1. 1,1'-Biphenyl-2-yl-alkylamin-Derivate der Formel (I)

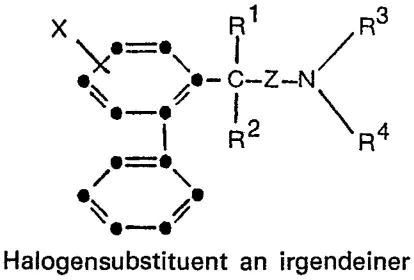

(I)

worin X Wasserstoff oder ein Halogensubstituent an irgendeiner Stellung des Phenylrings ist,

$R^1$ Wasserstoff, Hydroxy, C≡N, $CONR^5R^6$, worin $R^5$ und $R^6$ unabhängig, voneinander Wasserstoff oder $C_1$—$C_6$-Alkyl sind, oder $CO_2R^8$ bedeutet, worin $R^8$ für Wasserstoff oder $C_1$—$C_4$-Alkyl steht,
$R^2$ Wasserstoff oder $C_1$—$C_3$-Alkyl darstellt, Z für

$$-(CH_2)_n- \text{ oder } -CH_2\underset{}{\overset{OH}{|}}\!\!CHCH_2-$$

steht, worin n eine ganze Zahl von 2 bis 4 dedeutet,

$R^3$ und $R^4$ unabhängig für Wasserstoff, $C_1$—$C_6$-Alkyl, $CH_2$—$C_2$—$C_5$-Alkenyl oder Phenyl-$C_1$—$C_3$-alkyl stehen oder zusammen mit dem benachbarten Stickstoffatom eine heterocyclische Gruppe mit 5 bis 7 Kohlenstoffatomen bilden, die gegebenenfalls ein Heteroatom aus der Gruppe Sauerstoff oder Stickstoff enthält,
oder pharmazeutisch unbedenkliche Salze hiervon.

2. Verbindung der Formel (I) nach Anspruch 1, dadurch gekennzeichnet, daß X Wasserstoff oder Fluor, Chlor oder Brom in Stellung 5 des Biphenylylrings ist, $R^1$ Wasserstoff, Hydroxy, C≡N, $CONH_2$ oder COOH bedeutet, $R^2$ Wasserstoff oder Methyl ist, Z für $-(CH_2)_n-$ steht, falls n für 2 oder 3 steht, oder für $-CH_2CH(OH)CH_2-$ steht, falls $R^1$ Wasserstoff ist, und $R^3$ sowie $R^4$ unabhängig Wasserstoff, $C_1$—$C_6$-Alkyl, $CH_2$—$C_2$—$C_5$-Alkenyl oder Phenyl-$C_1$—$C_3$-alkyl sind oder zusammen mit dem benachbarten Stickstoffatom eine heterocyclische Gruppe aus der Gruppe Pyrrolidino, Piperidino und Morpholino bilden, oder ein pharmazeutisch unbedenkliches Säureadditionssalz oder ein quaternäres Ammoniumsalz hiervon.

3. Verbindung der Formel (I) nach Anspruch 1 oder 2, dadurch gekennzeichnet, daß $R^1$ für Wasserstoff steht.

4. Verbindung der Formel (I) nach einem der Ansprüche 1 bis 3, dadurch gekennzeichnet, daß $R^1$ für $CONH_2$ steht.

5. Verbindung der Formel (I) nach einem der vorhergehenden Ansprüche, dadurch gekennzeichnet, daß $R^3$ Wasserstoff ist und $R^4$ für Isopropyl steht.

6. Verbindung der Formel (I) nach einem der vorhergehenden Ansprüche, dadurch gekennzeichnet, daß Z für $-(CH_2)_n-$ steht, worin n für 3 steht.

7. N-Isopropyl-4-aminocarbonyl-4-(1,1'-biphenyl-2-yl)butylamin oder ein pharmazeutisch unbedenkliches Salz hiervon.

8. Verfahren zur Herstellung der Verbindungen der Formel (I) nach einem der Ansprüche 1 bis 7, dadurch gekennzeichnet, daß man

(a) ein Keton der Formel (II)

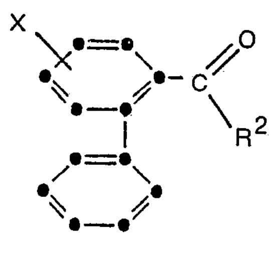

(II)

mit einem Grignard-Reagens der Formel

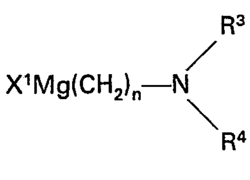

worin $X^1$ Chlor, Brom oder Iod ist und n für 3 oder 4 steht, umsetzt und hierdurch zu einer Verbindung der Formel (I) gelangt, worin $R^1$ Hydroxy bedeutet und Z für $-(CH_2)_n-$ steht, worin n für 2 bis 4 steht,

(b) eine Verbindung der Formel (II)

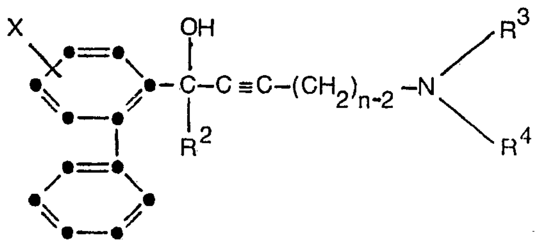

(II)

reduziert und hierdurch eine Verbindung der Formel (I) bildet, worin $R^1$ Hydroxy ist und Z für $—(CH_2)_n—$ steht, worin n für 3 oder 4 steht,

(c) ein Keton der Formel (III)

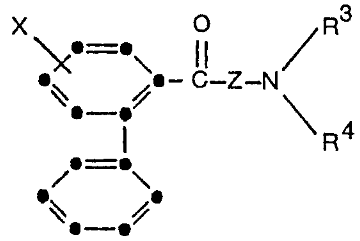

(III)

reduziert und hierdurch eine Verbindung der Formel (I) bildet, worin $R^1$ Hydroxy ist und $R^2$ Wasserstoff bedeutet,

(d) ein Nitril der Formel (IV)

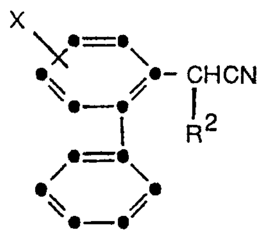

(IV)

mit einem Aminoalkylierungsmittel der Formel

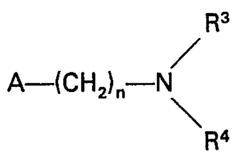

worin A eine abspaltbare Gruppe bedeutet, alkyliert und hierdurch eine Verbindung der Formel (I) bildet, worin $R^1$ für $—C{\equiv}N$ steht,

(e) ein Nitril der Formel (V)

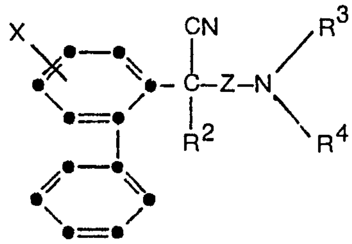

(V)

hydrolysiert und hierdurch eine Verbindung der Formel (I) bildet, worin $R^1$ für $—CONH_2$ oder $—COOH$ steht,

(f) ein Epoxid der Formel (VI)

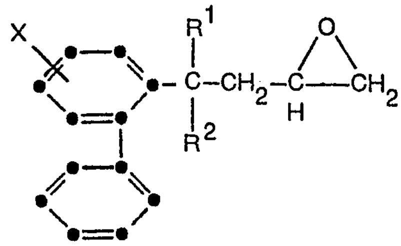

(VI)

mit einem Amin der Formel $HNR^3R^4$, worin $R^1$ die oben angegebene Bedeutung hat oder für eine geschützte Hydroxylgruppe steht, kondensiert und die Schutzgruppen gegebenenfalls entfernt, oder

(g) ein Olefin der Formel

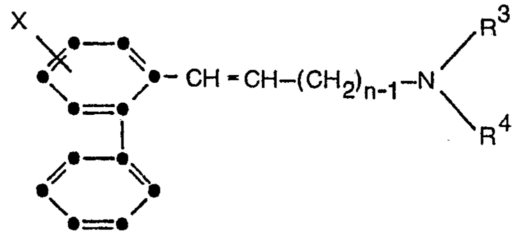

reduziert und hierdurch eine Verbindung der Formel (I) bildet, worin $R^1$ Wasserstoff ist und Z für $-(CH_2)_n-$ steht, worin n für 2 bis 4 steht.

9. Pharmazeutisch unbedenkliche Formulierung, dadurch gekennzeichnet, daß sie als Wirkstoff eine Verbindung der Formel (I) oder ein pharmazeutisch unbedenkliches Salz hiervon nach einem der Ansprüche 1 bis 7 in Verbindung mit einem pharmazeutisch unbedenklichen Träger hierfür enthält.

10. Verwendung einer Verbindung der Formel (I) oder eines pharmazeutisch unbedenklichen Salzes hiervon nach einem der Ansprüche 1 bis 7 zur Behandlung kardiovaskulärer Störungen.